(19) Europäisches Patentamt / European Patent Office / Office européen des brevets

(11) **EP 3 590 421 A1**

(12) **DEMANDE DE BREVET EUROPEEN**

(43) Date de publication:
08.01.2020 Bulletin 2020/02

(21) Numéro de dépôt: 19181383.1

(22) Date de dépôt: 19.06.2019

(51) Int Cl.:
*A61B 5/01* (2006.01)   *A61B 5/11* (2006.01)
*A61B 5/18* (2006.01)   *A61B 5/00* (2006.01)
*A61B 5/16* (2006.01)   *G16H 50/00* (2018.01)
*B60K 28/02* (2006.01)   *A61B 5/0402* (2006.01)
*A61B 5/0476* (2006.01)   *A61B 5/08* (2006.01)

(84) Etats contractants désignés:
AL AT BE BG CH CY CZ DE DK EE ES FI FR GB
GR HR HU IE IS IT LI LT LU LV MC MK MT NL NO
PL PT RO RS SE SI SK SM TR
Etats d'extension désignés:
BA ME
Etats de validation désignés:
KH MA MD TN

(30) Priorité: 02.07.2018 FR 1856073

(71) Demandeur: Airbus Opérations SAS
31060 Toulouse (FR)

(72) Inventeurs:
• VOLLARD-DERME, Cécile
31600 MURET (FR)
• BARROU, Laurent
31620 CASTELNAU D'ESTRETEFONDS (FR)

• MONFRAIX, Jean
31490 LEGUEVIN (FR)
• PAPAÏX, Benoît
31300 TOULOUSE (FR)
• DELPECH, Estelle
31830 PLAISANCE DU TOUCH (FR)
• REMCH, Hafeda
31100 TOULOUSE (FR)
• CHANEL, Guillaume
74160 SAINT JULIEN EN GENEVOIS (FR)
• LEWKOWICZ, Daniel
31400 TOULOUSE (FR)
• BOYER, Stanislas
31330 GRENADE (FR)

(74) Mandataire: Gevers & Orès
Immeuble le Palatin 2
3 Cours du Triangle
CS 80165
92939 Paris La Défense Cedex (FR)

(54) **PROCÉDÉ ET DISPOSITIF DE SURVEILLANCE DE LA CAPACITÉ À AGIR D'UN MEMBRE D'ÉQUIPAGE D'UN AÉRONEF**

(57) - Procédé et dispositif de surveillance de la capacité d'un membre d'équipage d'un aéronef.
- Le dispositif de surveillance (1) comprend au moins un module de mesure (2) d'au moins un paramètre physiologique du membre d'équipage, au moins un module de consolidation (3) du ou des paramètres physiologiques mesurés, un module de fusion (4) du ou des paramètres physiologiques consolidés afin de détecter au moins un statut physiologique du membre d'équipage, un module de filtrage (5) du ou des statuts physiologiques, un module de détermination (6) d'un niveau d'incapacité du membre de d'équipage, un module de transmission (7) d'un signal représentatif du niveau d'incapacité du membre d'équipage à un dispositif utilisateur (8).

Fig. 2

EP 3 590 421 A1

**Description**

DOMAINE TECHNIQUE

La présente invention concerne un procédé et un dispositif de surveillance de la capacité d'un membre d'équipage dans un aéronef.

ÉTAT DE LA TECHNIQUE

La capacité d'un membre d'équipage peut faire partie des critères permettant d'effectuer un vol dans de bonnes conditions.

La surveillance de la capacité d'un membre d'équipage permet de détecter une incapacité dudit membre d'équipage. Une incapacité est définie comme correspondant à une dégradation de l'état psychophysiologique du pilote. Cet état psychophysiologique est généralement dû à des événements médicaux psychologique ou physiologique qui ont été vécus par le membre d'équipage. L'incapacité peut se manifester sous diverses formes. Par exemple, elle peut se manifester soudainement et totalement sous la forme d'un infarctus ou plus subtilement et partiellement sous la forme d'un état de fatigue. L'incapacité d'un membre d'équipage ne peut être détectée que par le membre d'équipage lui-même ou par un autre membre d'équipage. Il en résulte qu'il est parfois difficile de détecter certaines incapacités d'un membre d'équipage.

Le document US 2017/332975 décrit un système d'évaluation des capacités d'action d'un individu.

EXPOSÉ DE L'INVENTION

La présente invention a pour objet de pallier ces inconvénients en proposant un procédé et un dispositif permettant de détecter une incapacité d'un membre d'équipage par la surveillance de la capacité du membre d'équipage.

À cet effet, l'invention concerne un procédé de surveillance de la capacité d'un membre d'équipage d'un aéronef.

Selon l'invention, le procédé comprend les étapes suivantes :

- une étape de mesure, mise en oeuvre par au moins un module de mesure, consistant à mesurer au moins un paramètre physiologique du membre d'équipage et à fournir au moins une note de confiance associée respectivement au ou aux modules de mesure ;
- une étape de consolidation, mise en oeuvre par au moins un module de consolidation, consistant à consolider le ou les paramètres physiologiques mesurés et à déterminer une ou des notes de confiance consolidées du ou des paramètres physiologiques consolidés ;
- une étape de fusion, mise en oeuvre par un module de fusion, consistant à fusionner le ou les paramètres physiologiques consolidés afin de détecter au moins un statut physiologique du membre d'équipage à partir d'au moins une fonction de détection de statut physiologique ;
- une étape de filtrage, mise en oeuvre par un module de filtrage, consistant à filtrer le ou les statuts physiologiques détectés dans l'étape de fusion afin de conserver le ou les statuts physiologiques les plus probables ;
- une étape de détermination, mise en oeuvre par un module de détermination, consistant à déterminer un niveau d'incapacité du membre de d'équipage à partir du ou des statuts physiologiques les plus probables déterminés dans l'étape de filtrage ;
- une étape de transmission, mise en oeuvre par un module de transmission, consistant à transmettre à un module utilisateur un signal représentatif du niveau d'incapacité du membre d'équipage.

Ainsi, grâce à l'invention, il est possible de détecter une incapacité du membre d'équipage grâce à la mesure de paramètres physiologiques afin qu'un plan d'action puisse être mis en oeuvre, si nécessaire.

Selon une première particularité, l'étape de fusion comprend des sous-étapes d'une première fonction de détection dont :

- une première sous-étape de comparaison, mise en oeuvre par un premier sous-module de comparaison, consistant à comparer au moins un paramètre physiologique consolidé par rapport à au moins un seuil d'incapacité prédéterminé ;
- une première sous-étape de détermination, mise en oeuvre par un premier sous-module de détermination, consistant à déterminer au moins un premier statut physiologique en fonction du résultat de la comparaison de la première sous-étape de comparaison.

Selon une deuxième particularité, l'étape de fusion comprend des sous-étapes d'une deuxième fonction de détection dont :

- une deuxième sous-étape de détermination, mise en oeuvre par un deuxième sous-module de détermination, consistant à déterminer au moins un deuxième statut physiologique à partir d'au moins un paramètre physiologique consolidé et d'un système d'inférence comprenant des règles conditionnelles et des densités de probabilités, les règles conditionnelles et les densités de probabilité étant basées sur des expériences médicales et des analyses de données médicales.

Selon une troisième particularité, l'étape de fusion comprend des sous-étapes d'une troisième fonction de détection dont :

- une première sous-étape de calcul, mise en oeuvre par un premier sous-module de calcul, consistant à calculer une probabilité de bonne santé, la probabilité de bonne santé correspondant à une probabilité de rencontrer le ou les paramètres physiologiques consolidés pour un membre d'équipage en bonne santé ;
- une deuxième sous-étape de comparaison, mise en oeuvre par un deuxième sous-module de comparaison, consistant à comparer la probabilité de bonne santé à au moins un seuil de bonne santé prédéterminé ;
- une troisième sous-étape de détermination, mise en oeuvre par un troisième sous-module de détermination, consistant à déterminer au moins un troisième statut physiologique en fonction du résultat de la comparaison de la deuxième sous-étape de comparaison.

De plus, l'étape de filtrage comprend les sous-étapes suivantes :

- une deuxième sous-étape de calcul, mise en oeuvre par un deuxième sous-module de calcul, consistant à calculer une moyenne de la ou des notes de confiance pour chacune des fonctions de détection de statut physiologique, la ou les notes de confiance étant associées au ou aux modules de mesure configurés pour mesurer le ou les paramètres physiologiques utilisés par la fonction de détection de statut physiologique ;
- une troisième sous-étape de comparaison, mise en oeuvre par un troisième sous-module de comparaison, consistant à comparer la moyenne calculée dans la deuxième sous-étape de calcul à un seuil de note de confiance prédéterminé ;
- une quatrième sous-étape de détermination, mise en oeuvre par un quatrième sous-module de détermination, consistant à déterminer le ou les statuts physiologiques les plus probables en fonction du résultat de la comparaison de la troisième sous-étape de comparaison.

Selon un mode de réalisation, l'étape de mesure comprend les sous-étapes suivantes :

- une sous-étape de mesure de fatigue, mise en oeuvre par un premier module de mesure de fatigue disposé dans un équipement de tête configuré pour être coiffé par le membre d'équipage et par un deuxième module de mesure de fatigue disposé dans un premier équipement vidéo configuré pour acquérir des images du membre d'équipage, consistant à acquérir des mesures de fatigue du membre d'équipage ;
- une sous-étape de mesure de rythme cardiaque, mise en oeuvre par un premier module de mesure de rythme cardiaque disposé dans l'équipement de tête, un deuxième module de mesure de rythme cardiaque disposé dans le premier équipement vidéo et un troisième module de mesure de rythme cardiaque disposé dans un siège configuré pour recevoir le membre d'équipage, consistant à acquérir des mesures de rythme cardiaque du membre d'équipage ;
- une sous-étape de mesure de température corporelle, mise en oeuvre par un premier module de mesure de température corporelle disposé dans l'équipement de tête, un deuxième module de mesure de température corporelle disposé dans le premier équipement vidéo et un troisième module de mesure de température corporelle disposé dans le siège, consistant à acquérir des mesures de température corporelle du membre d'équipage ;
- une sous-étape de mesure d'orientation de tête, mise en oeuvre par un premier module de mesure d'orientation de tête disposé dans l'équipement de tête et un deuxième module de mesure d'orientation de tête disposé dans le premier équipement vidéo, consistant à acquérir des mesures d'orientation de tête du membre d'équipage ;
- une sous-étape de mesure de mouvement de tête, mise en oeuvre par un premier module de mesure de mouvement de tête disposé dans l'équipement de tête et un deuxième module de mesure de mouvement de tête disposé dans le premier équipement vidéo, consistant à acquérir des mesures de mouvement de tête du membre d'équipage ;
- une sous-étape de mesure de fréquence de clignement oculaire, mise en oeuvre par un module de mesure oculaire disposé dans le premier équipement vidéo, consistant à acquérir des mesures de fréquence de clignement oculaire du membre d'équipage ;
- une sous-étape de mesure de présence, mise en oeuvre par un module de mesure de présence disposé dans le premier équipement vidéo, consistant à acquérir des mesures de présence du membre d'équipage ;
- une sous-étape de mesure de mouvement, mise en oeuvre par un module de mesure de mouvement disposé dans le siège, consistant à acquérir des mesures de mouvement du membre d'équipage.

De façon avantageuse, la sous-étape de mesure de fatigue est en outre mise en oeuvre par un troisième module de mesure de fatigue disposé dans un siège configuré pour recevoir le membre d'équipage.

De plus, l'étape de consolidation comprend les sous-étapes suivantes :

- une sous-étape de consolidation de la fatigue mesurée, mise en oeuvre par un premier sous-module de consolidation, consistant à déterminer une mesure de fatigue consolidée à partir des mesures de fatigue acquises dans la sous-étape de mesure de fatigue ;

- une sous-étape de consolidation du rythme cardiaque mesuré, mise en oeuvre par un deuxième sous-module de consolidation, consistant à déterminer une mesure de rythme cardiaque consolidée à partir des mesures de rythme cardiaque acquises dans la sous-étape de mesure de rythme cardiaque ;

- une sous-étape de consolidation de la température corporelle mesurée, mise en oeuvre par un troisième sous-module de consolidation, consistant à déterminer une mesure de température corporelle consolidée à partir des mesures de température corporelle acquises dans la sous-étape de mesure de température corporelle ;

- une sous-étape de consolidation de l'orientation de tête mesurée, mise en oeuvre par un quatrième sous-module de consolidation, consistant à déterminer une mesure d'orientation de tête consolidée à partir des mesures d'orientation de tête acquises dans la sous-étape de mesure d'orientation de tête ;

- une sous-étape de consolidation du mouvement de tête mesuré, mise en oeuvre par un cinquième sous-module de consolidation, consistant à déterminer une mesure de mouvement de tête consolidée à partir des mesures de mouvement de tête acquises dans la sous-étape de mesure de mouvement de tête.

En outre, les sous-étapes de la première fonction de détection sont mises en oeuvre pour le paramètre physiologique consolidé correspondant à

la mesure de fatigue consolidée,

le premier statut physiologique correspondant à un statut de fatigue,

la mesure de fatigue consolidée étant comparée à un premier seuil d'incapacité prédéterminé dans la première sous-étape de comparaison.

Par ailleurs, les sous-étapes de la première fonction de détection sont mises en oeuvre pour les paramètres physiologiques consolidés correspondant à la mesure de température corporelle consolidée et à la mesure de rythme cardiaque consolidée,

les premiers statuts physiologiques correspondant à un statut fébrile et à un statut cardiaque,

la mesure de température corporelle consolidée étant comparée à un deuxième seuil d'incapacité prédéterminé dans la première sous-étape de comparaison,

la mesure de rythme cardiaque consolidée étant comparée à un troisième seuil d'incapacité prédéterminé dans la première sous-étape de comparaison.

De plus, les sous-étapes de la troisième fonction de détection sont mises en oeuvre pour les paramètres physiologiques consolidés correspondant à la mesure de température corporelle consolidée et à la mesure de rythme cardiaque consolidée,

les troisièmes statuts physiologiques correspondant à un statut fébrile et à un statut cardiaque,

la probabilité de bonne santé pour la mesure de température corporelle consolidée étant comparée à un premier seuil de bonne santé prédéterminé dans la deuxième sous-étape de comparaison,

la probabilité de bonne santé pour la mesure de rythme cardiaque consolidée étant comparée à un deuxième seuil de bonne santé prédéterminé dans la deuxième sous-étape de comparaison.

De plus, les sous-étapes de la deuxième fonction de détection sont mise en oeuvre pour les paramètres physiologiques consolidés correspondant à la mesure d'orientation de tête consolidée, à la mesure de mouvement de tête consolidée, à la mesure de présence consolidée et à la mesure de fréquence de clignement oculaire consolidée,

le deuxième statut physiologique correspondant à un premier statut de niveau de conscience.

De plus, les sous-étapes de la troisième fonction de détection sont mises en oeuvre pour les paramètres physiologiques consolidés correspondant à la mesure d'orientation de tête consolidée, à la mesure de mouvement de tête consolidée, à la mesure de présence consolidée et à la mesure de fréquence de clignement oculaire consolidée,

le troisième statut physiologique correspondant à un deuxième statut de niveau de conscience,

une première probabilité de bonne santé globale étant déterminée dans la première sous-étape de calcul à partir d'une probabilité de bonne santé pour la mesure de d'orientation de tête consolidée, d'une probabilité de bonne santé pour la mesure de mouvement de tête consolidée, d'une probabilité de bonne santé pour la mesure de présence consolidée et d'une probabilité de bonne santé pour la mesure de fréquence de clignement oculaire consolidée, la première probabilité de bonne santé globale étant comparée dans la deuxième sous-étape de comparaison à un premier seuil de bonne santé globale prédéterminé.

De plus, les sous-étapes de la troisième fonction de détection sont mises en oeuvre pour les paramètres physiologiques consolidés correspondant à la mesure de fatigue, à la mesure de température corporelle, à la mesure de rythme cardiaque, à la mesure d'orientation de tête consolidée, à la mesure de mouvement de tête consolidée, à la mesure de présence consolidée, à la mesure de fréquence de clignement oculaire consolidée et à la mesure de mouvement du membre d'équipage, le troisième statut physiologique correspondant à un statut d'incapacité du membre d'équipage,

une deuxième probabilité de bonne santé globale est déterminée dans la première sous-étape de calcul à partir d'une probabilité de bonne santé pour la mesure de température corporelle consolidée, d'une probabilité de bonne santé pour la mesure de rythme cardiaque consolidée, d'une probabilité de bonne santé pour la mesure de d'orientation de tête consolidée, d'une probabilité de bonne santé pour la mesure de mouvement de tête consolidée, d'une probabilité de bonne santé pour la mesure de présence consolidée, d'une probabilité de bonne santé pour la mesure de fréquence de clignement oculaire consolidée et d'une probabilité de bonne santé pour la mesure de mouvement du membre d'équipage consolidée,

la deuxième probabilité de bonne santé globale étant comparée dans la deuxième étape de comparaison à un deuxième seuil de bonne santé globale prédéterminé.

L'invention concerne également un dispositif de surveillance de la capacité d'un membre d'équipage d'un aéronef.

Selon l'invention, le dispositif comprend :

- au moins un module de mesure, configuré pour mesurer au moins un paramètre physiologique du membre d'équipage et à fournir au moins une note de confiance associée respectivement au ou aux modules de mesure ;
- au moins un module de consolidation, configuré pour consolider le ou les paramètres physiologiques mesurés et à déterminer la ou les notes de confiance consolidées du ou des paramètres physiologiques consolidés ;
- un module de fusion, configuré pour fusionner le ou les paramètres physiologiques consolidés afin de détecter au moins un statut physiologique du membre d'équipage à partir d'au moins une fonction de détection de statut physiologique ;
- un module de filtrage, configuré pour filtrer le ou les statuts physiologiques détectés par le module de fusion afin de conserver le ou les statuts physiologiques les plus probables ;
- un module de détermination, configuré pour déterminer un niveau d'incapacité du membre de d'équipage à partir du ou des statuts physiologiques les plus probables déterminés par le module de filtrage ;
- un module de transmission, configuré pour transmettre à un module utilisateur un signal représentatif du niveau d'incapacité du membre d'équipage.

L'invention concerne également un aéronef, en particulier un avion de transport, qui comporte un dispositif de surveillance de la capacité d'un membre d'équipage, tel que spécifié ci-dessus.

BRÈVE DESCRIPTION DES FIGURES

L'invention, avec ses caractéristiques et avantages, ressortira plus clairement à la lecture de la description faite en référence aux dessins annexés dans lesquels :

- la figure 1 représente un mode de réalisation du dispositif de surveillance,
- la figure 2 représente un mode de réalisation du procédé de surveillance,
- la figure 3 représente un aéronef embarquant le dispositif de surveillance.

DESCRIPTION DÉTAILLÉE

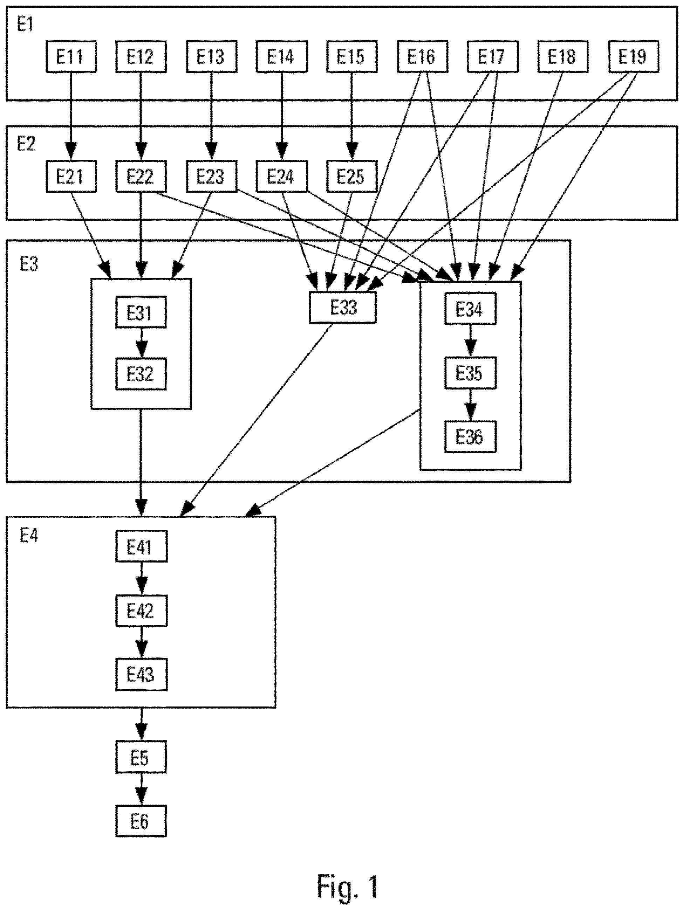

Le dispositif de surveillance 1 de la capacité d'un membre d'équipage d'un aéronef AC est représenté sur la figure 1. Dans la suite de la description, le dispositif de surveillance 1 de la capacité d'un membre d'équipage d'un aéronef AC sera appelé « dispositif de surveillance ». Le membre d'équipage peut correspondre au pilote de l'aéronef AC.

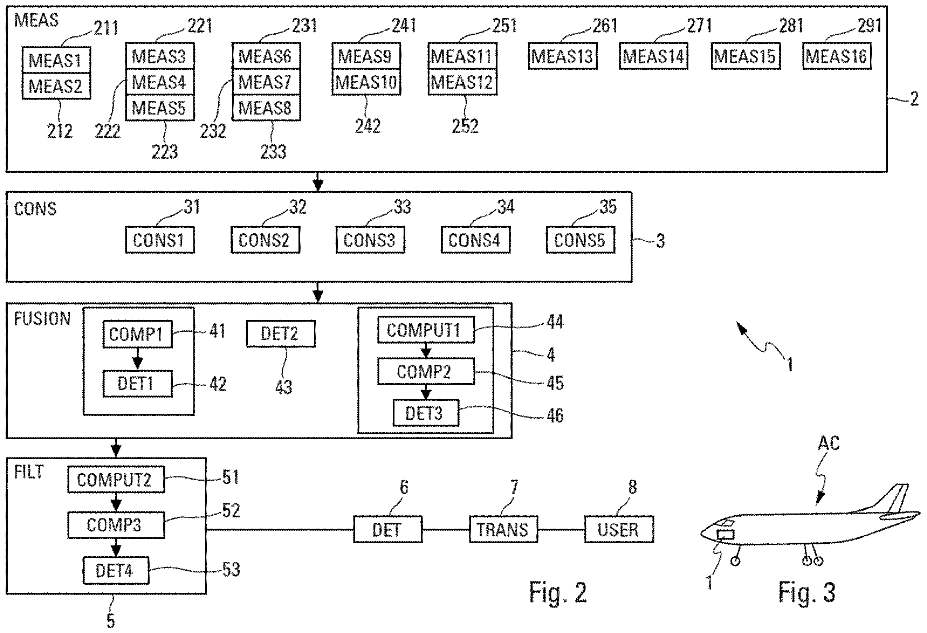

Le dispositif de surveillance 1, embarqué sur l'aéronef AC (figure 3), comprend au moins un module de mesure MEAS (MEAS pour « measurement module » en anglais) 2. Le ou les modules de mesure sont configurés pour mesurer au moins un paramètre physiologique du membre d'équipage et à fournir au moins une note de confiance associée respectivement au ou aux modules de mesure.

Selon un mode de réalisation préféré, le module de mesure comprend un ensemble de modules de mesure 2 répartis dans un équipement de tête (« boomset » en anglais) configuré pour être coiffé par le membre d'équipage, dans un siège configuré pour recevoir le membre d'équipage et dans un équipement vidéo configuré pour acquérir des images du membre d'équipage.

Dans ce mode de réalisation préféré, l'équipement de tête comprend un module de mesure de fatigue MEAS1 211, un module de mesure de rythme cardiaque MEAS3 221, un module de mesure de température corporelle MEAS6 231, un module de mesure d'orientation de tête MEAS9 241, un module de mouvement de tête MEAS11 251 et, éventuellement un module de mesure de rythme respiratoire.

Le module de mesure de rythme cardiaque 221 est monté sur l'équipement de tête de façon qu'il soit situé sur une région du front ou une région des tempes du membre d'équipage coiffant l'équipement de tête. Avantageusement, le module de mesure de rythme cardiaque 221 utilise le principe de la pléthysmographie optique (« photoplethysmography » en anglais).

Le module de mesure de fatigue 211 comprend des sondes d'électroencéphalogramme et une unité de calcul de niveau de fatigue. Les sondes sont configurées pour mesurer l'activité électrique du cerveau du membre d'équipage, en particulier les ondes bêta, alpha, thêta et delta. Par exemple, deux sondes sont montées dans le casque de façon que la première sonde, dite sonde Cz, se situe sur le vertex du crâne du membre d'équipage coiffant l'équipement de tête et que la deuxième sonde, dite sonde Pz, se situe sur le crâne à 10 cm environ en arrière du vertex. D'autres régions peuvent être couvertes selon les mesures souhaitées. Avantageusement, les sondes sont des sondes sèches pour faciliter l'utilisation de l'équipement de tête et pour éviter l'utilisation de produits consommables tels que des mousses ou des gels. L'unité de calcul permet de déterminer, grâce à un algorithme, le niveau de fatigue du membre d'équipage coiffant l'équipement de tête à partir des données fournies par les sondes.

Le module de mesure de température corporelle 231 comprend un premier capteur monté sur l'équipement de tête de façon qu'il soit disposé dans la région située derrière le lobe de l'oreille du membre d'équipage coiffant l'équipement de tête. En effet, cette région assure une grande stabilité de la mesure de la température corporelle car cette région est peu sujette à des variations de température. Le module de mesure de température corporelle 231 peut également être monté sur l'équipement de tête de façon qu'il soit situé dans la région des tempes du membre d'équipage coiffant l'équipement de tête. Avantageusement, le module de mesure de température corporelle 231 utilise le principe de la réflectométrie à infrarouge. Étant donné que les mesures réalisées avec ce principe sont dépendantes de la distance entre le capteur et la peau du membre d'équipage, un adaptateur mécanique peut être utilisé pour assurer un ajustement constant de la distance quand l'équipement de tête est coiffé. Le module de mesure de température corporelle 231 peut également comprendre un deuxième capteur de température ambiante. La mesure de température ambiante permet d'ajuster les mesures de température du premier capteur afin d'obtenir une mesure de température corporelle plus précise.

Le module de mesure d'orientation de tête 241 et le module de mouvement de tête 251 forment une unité de mesure inertielle. L'unité de mesure inertielle présente dix degrés de liberté grâce à la combinaison d'un gyromètre, d'un accéléromètre et d'un magnétomètre en fournissant l'attitude la tête selon les axes de tangage, de roulis et de lacet, l'accélération linéaire de la tête selon les trois axes et la vitesse linéaire de la tête selon les trois axes. L'orientation de tête et les mouvements de tête peuvent alors en être déduits.

Dans le mode de réalisation préféré, l'équipement vidéo comprend un module de mesure oculaire MEAS13 261, un module de mesure de fatigue MEAS2 212, un module de mesure de rythme cardiaque MEAS4 222, un module d'orientation de tête MEAS10 242, un module de mesure de température corporelle MEAS7 232, un module de mouvement de tête MEAS12 252, un module de mesure de présence MEAS14 271 et, éventuellement un module de mesure de normalité MEAS16 291. L'équipement vidéo peut également comprendre un module de mesure de rythme respiratoire.

Une mesure de normalité peut correspondre à une mesure représentative de la normalité d'une scène captée par un capteur vidéo.

L'équipement vidéo peut comprendre au moins une caméra et un éclairage à infrarouge afin d'améliorer les capacités de la détection. Avantageusement, la ou les caméras sont disposées afin que la ou les caméras puissent enregistrer au moins les yeux du membre d'équipage dans différentes configurations de l'équipage. Les modules de l'équipement vidéo est configuré pour mesurer différents paramètres relatifs au membre d'équipage.

Par exemple, le module de mesure oculaire 261 permet de mesurer au moins l'un des paramètres suivants :

- le regard,

- des mesures liées à l'oeil et des parties de l'oeil (iris, pupille, etc.) telles que la fermeture de l'oeil, clignement, diamètre, etc.,

- l'expression du visage (joie, anxiété, tristesse, etc.).

Le module de mesure d'orientation de tête 242 peut mesurer la position de la tête (angles d'Euler).
Le module de mesure de mouvement de tête 252 peut mesurer les mouvements de la tête et les bâillements.
Le module de mesure de rythme cardiaque 222 peut mesurer le rythme cardiaque du membre d'équipage.
Le module de mesure de température corporelle 232 peut mesurer la température corporelle du membre d'équipage.
Le module de mesure de présence 271 peut mesurer la présence ou non du membre d'équipage sur son siège, ou bien dans la cabine de pilotage (« cockpit » en anglais) mais non dans son siège, ou bien hors de la cabine de pilotage.
Le module de mesure de fatigue 212 peut comprendre une unité d'analyse de données vidéo. Cette unité d'analyse de données vidéo permet, par exemple, de déterminer un niveau de fatigue du membre d'équipage grâce à un algorithme configuré pour calculer des paramètres tels qu'un pourcentage de fermeture de paupière sur la pupille dans le temps (PERCLOS pour « PERcentage of eyelid CLOSure over the pupil over time » en anglais).
Le module de rythme respiratoire peut comprendre une autre unité d'analyse de données vidéo pour déterminer la mesure de rythme respiratoire. Les données vidéo peuvent être fournies par une caméra infrarouge. Le siège peut également comprendre au moins un module de mesure du rythme respiratoire.
Les modules de l'équipement vidéo sont aptes à fonctionner sous différentes conditions d'éclairage de la cabine de pilotage (de jour, de nuit, au crépuscule, etc.) et sous différentes conditions d'éclairage du visage du membre d'équipage (éclairé à moitié, éclairé horizontalement ou verticalement). Le module de mesure oculaire 261 peut également fonctionner normalement pour des membres d'équipage portant tout type de lunettes de soleil.
Dans le mode de réalisation préféré, le siège comprend un module de mesure de rythme cardiaque MEAS5 223, un module de mesure de température corporelle MEAS8 233, un module de mesure de mouvement MEAS15 281 (mouvement corporel). Le siège peut également comprendre au moins un module de mesure de fatigue (non représenté).

Les modules compris dans le siège peuvent comprendre des capteurs disposés à des endroits adéquats pour mesurer le rythme cardiaque, le rythme de respiration, la température corporelle, la posture du membre d'équipage, les mouvements corporels du membre d'équipage et sa présence.

Par exemple, le module de mesure de rythme cardiaque 223 comprend des capteurs de type électrocardiogramme utilisant des électrodes pour le rythme cardiaque, la révolution cardiaque le rythme respiratoire et l'actimétrie. En ce qui concerne la mesure du rythme respiratoire (de respiration), un capteur peut être intégré dans le siège en utilisant, par exemple, une technique de balistico-cardiographie (BCG pour « ballistocardiography » en anglais) ou d'électrocardiographie capacitive (c-ECG pour « capacitive electrocardiography » en anglais).

Le module de mesure de température corporelle 233 peut comprendre des capteurs de température tels que des thermocouples, des capteurs de détection de résistance électrique, ou des capteurs de température basés sur la bande interdite du silicium (« silicon bandgap temperature sensor » en anglais).

Les capteurs peuvent être disposés à différents endroits tels que :

- au sommet de coussinets du siège,
- intégrés dans une housse du siège,
- sous la housse du siège,
- intégrés dans la mousse des coussinets,
- sous les coussinets.

Un premier module de mesure de fatigue compris dans le siège peut utiliser des capteurs de pression permettant de déterminer une attitude posturale du membre d'équipage. Un deuxième module de mesure de fatigue peut être intégré dans un appui-tête du siège. Ce deuxième module de mesure de fatigue peut utiliser un capteur d'électroencéphalogramme (EEG pour « Electroencephalogram » en anglais).

Le dispositif de surveillance 1 comprend en outre au moins un module de consolidation CONS (CONS pour « consolidation module » en anglais) 3. Le module de consolidation 3 est configuré pour consolider le ou les paramètres physiologiques mesurés et pour déterminer la ou les notes de confiance consolidées du ou des paramètres physiologiques consolidés.

Le module de consolidation 3 permet d'assurer une robustesse et une fiabilité des informations obtenues à partir des mesures, afin de pallier la défaillance d'un capteur ou l'inexactitude d'un capteur.

Une fonction de consolidation se présente sous la forme suivante :

$$C(\langle s_1, cs_1 \rangle, \ldots, \langle s_n, cs_n \rangle) = \langle S, CS \rangle,$$

dans laquelle :

- $s_i$ correspond à la mesure fournie par un capteur i,
- $cs_1$ correspond la note de confiance de la mesure fournie par le capteur i,
- $S$ correspond à la mesure consolidée,
- $CS$ correspond à la note de confiance de la mesure consolidée.

Chaque fonction de consolidation peut être obtenue automatiquement en utilisant des techniques d'apprentissage automatique (« machine learning » en anglais), telles que les réseaux neuronaux ou les arbres de décision. Elle peut prendre la forme d'une combinaison linéaire (ou autre), de règles conditionnelles, d'un graphe neuronal contenant des fonctions d'activation, etc.

Selon le mode de réalisation préféré, le module de consolidation 3 comprend les sous-modules suivants :

- un sous-module de consolidation CONS1 31 configuré pour déterminer une mesure de fatigue consolidée à partir des mesures de fatigue acquises par les modules de mesure de fatigue 211, 212,
- un sous-module de consolidation CONS2 32 configuré pour déterminer une mesure de rythme cardiaque consolidée à partir des mesures de rythme cardiaque acquises par les modules de mesure de rythme cardiaque 221, 222, 223,
- un sous-module de consolidation CONS3 33 configuré pour déterminer une mesure de température corporelle consolidée à partir des mesures de température corporelle acquises par les modules de mesure de température corporelle 231, 232, 233,
- un sous-module de consolidation CONS4 34 configuré pour déterminer une mesure d'orientation de tête consolidée à partir des mesures d'orientation de tête acquises par les modules de mesure d'orientation de tête 241, 242,
- un sous-module de consolidation CONS5 35 configuré pour déterminer une mesure de mouvement de tête consolidée à partir des mesures de mouvement de tête acquises par les modules de mesure de mouvement de tête 251, 252.

Le dispositif de surveillance 1 comprend également un module de fusion FUSION 4 configuré pour fusionner le ou les paramètres physiologiques consolidés afin de détecter au moins un statut physiologique du membre d'équipage à partir d'au moins une fonction de détection de statut physiologique.

Le ou les statuts physiologiques peuvent correspondre à un ou des états physiologiques ou à un ou des états psychiques et physiologiques du membre d'équipage. Par exemple, les statuts physiologiques peuvent correspondre à un état de fatigue, un état fiévreux, un état cardiaque ou un état de niveau de conscience.

Le ou les statuts physiologiques peuvent prendre des valeurs binaires indiquant l'état physiologiques du membre d'équipage. Par exemple, un statut physiologique correspondant à un état de fatigue prenant une valeur binaire égale à 1 indique que le membre d'équipage est considéré comme étant fatigué. Si ce statut physiologique prend une valeur égale à 0, cela indique que le membre d'équipage n'est pas considéré comme étant fatigué.

Le module de fusion 4 peut comprendre des sous-modules mettant en oeuvre une première fonction de détection dont :

- un sous-module de comparaison COMP1 (COMP pour « comparison module » en anglais) 41 configuré pour comparer au moins un paramètre physiologique consolidé par rapport à au moins un seuil d'incapacité prédéterminé ;
- un sous-module de détermination DET1 (DET pour « détermination module » en anglais) 42 configuré pour déterminer au moins un premier statut physiologique en fonction du résultat de la comparaison obtenu par le sous-module de comparaison 41.

Le ou les seuils d'incapacité prédéterminés correspondent à des seuils d'expert sur la base d'expériences médicales et d'analyses de données médicales. Leur but est de déterminer quand un paramètre physiologique consolidé présente une valeur indiquant un symptôme d'une incapacité donnée.

La première fonction de détection peut se présenter sous la forme suivante :

$$T(\langle p, cs \rangle) = P,$$

dans laquelle :

- $p$ correspond au paramètre physiologique,

- $cs$ correspond à la note de confiance du paramètre physiologique,

- $P$ correspond au statut physiologique du membre d'équipage.

Selon le mode de réalisation préféré, la première fonction de détection est mise en oeuvre pour le paramètre physiologique consolidé correspondant à la mesure de fatigue consolidée. Le premier statut physiologique correspond à un statut de fatigue. La mesure de fatigue consolidée est comparée à un premier seuil d'incapacité prédéterminé dans le sous-module de comparaison 41.

Par exemple, si la mesure de fatigue consolidée est supérieure ou égale au premier seuil d'incapacité prédéterminé, le statut de fatigue prend une valeur égale à 1, indiquant que le membre d'équipage est considéré comme fatigué. Si la mesure est inférieure au premier seuil d'incapacité prédéterminé, le statut de fatigue prend une valeur égale à 0, indiquant que le membre d'équipage est considéré comme non fatigué.

Selon le mode de réalisation préféré, la première fonction de détection est également mise en oeuvre pour les paramètres physiologiques consolidés correspondant à la mesure de température corporelle consolidée et à la mesure de rythme cardiaque consolidée. Les premiers statuts physiologiques correspondent à un statut fébrile et à un statut cardiaque. La mesure de température corporelle consolidée est comparée à un deuxième seuil d'incapacité prédéterminé par le sous-module de comparaison 41. La mesure de rythme cardiaque consolidée est comparée à un troisième seuil d'incapacité prédéterminé par le sous-module de comparaison 41.

Par exemple, si la mesure de température corporelle consolidée est supérieure ou égal au deuxième seuil d'incapacité prédéterminé, le statut fébrile prend une valeur égale à 1, indiquant que le membre d'équipage est considéré comme fiévreux. Si la mesure est inférieure au deuxième seuil d'incapacité prédéterminé, le statut fébrile prend une valeur égale à 0, indiquant que le membre d'équipage est considéré comme non fiévreux. Si la mesure de rythme cardiaque consolidée est supérieure ou égal au troisième seuil d'incapacité prédéterminé, le statut cardiaque prend une valeur égale à 1, indiquant que le membre d'équipage est considéré comme ayant un problème cardiaque. Si la mesure est inférieure au troisième seuil d'incapacité prédéterminé, le statut cardiaque prend une valeur égale à 0, indiquant que le membre d'équipage est considéré comme n'ayant pas de problème cardiaque.

Le module de fusion 4 peut comprendre des sous-modules mettant en oeuvre une deuxième fonction de détection dont :

- un sous-module de détermination DET2 43 configuré pour déterminer au moins un deuxième statut physiologique à partir d'au moins un paramètre physiologique consolidé et d'un système d'inférence.

Le but de cette deuxième fonction est de combiner des règles d'expert déclarées indépendamment afin de savoir si l'ensemble commun des paramètres physiologiques peut être considéré comme étant les symptômes d'une incapacité donnée.

La deuxième fonction de détection peut se présenter sous la forme suivante :

$$C(\langle p_1, cs_1 \rangle, \ldots, \langle p_n, cs_n \rangle) = P,$$

dans laquelle :

- $p_i$ correspond au paramètre physiologique i,

- $cs_i$ correspond à la note de confiance du paramètre physiologique i,

- $P$ correspond au statut physiologique du membre d'équipage.

Le système d'inférence correspond à un système d'expert, tel qu'un réseau bayésien ou une machine d'inférence, qui comprend des règles conditionnelles et des densités de probabilités. Les règles conditionnelles et les densités de probabilité sont basées sur des expériences médicales et des analyses de données médicales.

Selon le mode de réalisation préférée, la deuxième fonction de détection est mise en oeuvre pour les paramètres physiologiques consolidés correspondant à la mesure d'orientation de tête consolidée, à la mesure de mouvement de tête consolidée, à la mesure de présence consolidée, à la mesure de fréquence de clignement oculaire consolidée et, éventuellement, à la mesure de normalité consolidée. Le deuxième statut physiologique correspond à un premier statut de niveau de conscience.

Par exemple, le statut de niveau de conscience prend une valeur égale à 1 si la mesure d'orientation de tête consolidée, la mesure de mouvement de tête consolidée, la mesure de présence consolidée, la mesure de fréquence de clignement oculaire consolidée et, éventuellement, la mesure de normalité consolidée présentent des valeurs indiquant une perte de conscience par le système d'inférence. Le statut de niveau de conscience prend une valeur égale à 0 si la mesure d'orientation de tête consolidée, la mesure de mouvement de tête consolidée, la mesure de présence consolidée, la mesure de fréquence de clignement oculaire consolidée et, éventuellement, la mesure de normalité consolidée présentent des valeurs présentent des valeurs indiquant une non perte de conscience par le système d'inférence.

Le module de fusion 4 peut comprendre des sous-modules mettant en oeuvre une troisième fonction de détection dont :

- un sous-module de calcul COMPUT1 (COMPUT pour « computation module » en anglais) 44 configuré pour calculer une probabilité de bonne santé, la probabilité de bonne santé correspondant à une probabilité de rencontrer le ou les paramètres physiologiques consolidés pour un membre d'équipage en bonne santé ;
- un sous-module de comparaison COMP2 45 configuré pour comparer la probabilité de bonne santé à au moins un seuil de bonne santé prédéterminé ;
- un sous-module de détermination DET2 46 configuré pour déterminer au moins un troisième statut physiologique en fonction du résultat de la comparaison obtenu par le sous-module de comparaison 45.

La troisième fonction de détection correspond à une fonction de détection d'anomalie machine. La but de cette fonction est de savoir si un ensemble d'un ou de plusieurs paramètres physiologiques porte des valeurs anormales, ce qui veut dire que cet ensemble peut être considéré comme étant les symptômes d'une incapacité.

La troisième fonction de détection peut se présenter sous la forme suivante :

$$I(\langle p_1, cs_1 \rangle, ..., \langle p_n, cs_n \rangle) = p,$$

dans laquelle :

- $p_i$ correspond au paramètre physiologique i,

- $cs_i$ correspond à la note de confiance du paramètre physiologique i,

- $p$ correspond à la probabilité de bonne santé.

La troisième fonction peut être obtenue automatiquement en utilisant des techniques de modèles statistiques ou des techniques d'apprentissage automatique.

Le seuil de bonne santé prédéterminé peut être obtenu par un jugement d'expert.

Selon le mode de réalisation préféré, la troisième fonction de détection est mise en oeuvre pour les paramètres physiologiques consolidés correspondant à la mesure de température corporelle consolidée et à la mesure de rythme cardiaque consolidée. Les troisièmes statuts physiologiques correspondent à un statut fébrile et à un statut cardiaque. La probabilité de bonne santé pour la mesure de température corporelle consolidée est comparée à un premier seuil de bonne santé prédéterminé par le sous-module de comparaison 45. La probabilité de bonne santé pour la mesure de rythme cardiaque consolidée est comparée à un deuxième seuil de bonne santé prédéterminé par le sous-module de comparaison 45.

Par exemple, si la probabilité de bonne santé pour la mesure de température corporelle est inférieure ou égale au premier seuil de bonne santé prédéterminé, le statut fébrile prend une valeur égale à 1, indiquant que le membre d'équipage est considéré comme fiévreux. Si ladite probabilité est supérieure au premier seuil de bonne santé prédéterminé, le statut fébrile prend une valeur égale à 0, indiquant que le membre d'équipage est considéré comme non fiévreux. Si probabilité de bonne santé pour la mesure de rythme cardiaque consolidée est inférieure ou égale au deuxième seuil de bonne santé prédéterminé, le statut cardiaque prend une valeur égale à 1, indiquant que le membre d'équipage est considéré comme ayant un problème cardiaque. Si ladite probabilité est supérieure au deuxième seuil de bonne santé prédéterminé, le statut cardiaque prend une valeur égale à 0, indiquant que le membre d'équipage est considéré comme n'ayant pas de problème cardiaque.

De même, selon le mode de réalisation préféré, la troisième fonction de détection est mise en oeuvre pour les paramètres physiologiques consolidés correspondant à la mesure d'orientation de tête consolidée, à la mesure de mouvement de tête consolidée, à la mesure de présence consolidée, à la mesure de fréquence de clignement oculaire consolidée et, éventuellement, à la mesure de normalité consolidée. Le troisième statut physiologique correspond à un deuxième statut de niveau de conscience.

Selon une première variante, les probabilités de bonne santé sont calculées pour chacune des mesures par le sous-module de calcul 44. La probabilité de bonne santé pour la mesure de d'orientation de tête consolidée est comparée à un troisième seuil de bonne santé prédéterminé par le sous-module de comparaison 45. La probabilité de bonne santé pour la mesure de mouvement de tête consolidée est comparée à un quatrième seuil de bonne santé prédéterminé dans le sous-module de comparaison 45. La probabilité de bonne santé pour la mesure de présence consolidée est comparée à un cinquième seuil de bonne santé prédéterminé par le sous-module de comparaison 45. La probabilité de bonne santé pour la mesure de fréquence de clignement oculaire consolidée est comparée à un sixième seuil de bonne santé prédéterminé par le sous-module de comparaison 45. Éventuellement, la probabilité de bonne santé pour la mesure de normalité consolidée est comparée à un septième seuil de bonne santé prédéterminé dans le sous-module de comparaison 45.

Par exemple, le statut de niveau de conscience prend une valeur égale à 1, indiquant une perte de conscience du membre d'équipage :

- si la probabilité de bonne santé pour la mesure de d'orientation de tête consolidée est inférieure ou égale au troisième seuil de bonne santé, et

- si la probabilité de bonne santé pour la mesure de mouvement de tête consolidée est inférieure ou égale au quatrième seuil de bonne santé, et

- si la probabilité de bonne santé pour la mesure de présence consolidée est inférieure ou égale au cinquième seuil de bonne santé prédéterminé, et

- si la probabilité de bonne santé pour la mesure de fréquence de clignement oculaire consolidée inférieure ou égale au sixième seuil de bonne santé prédéterminé, et

- éventuellement, si la probabilité de bonne santé pour la mesure de normalité consolidée est inférieure ou égale au septième seuil de bonne santé prédéterminé.

Sinon, le statut de niveau de conscience prend une valeur égale à 0, indiquant une non perte de conscience du membre d'équipage.

Selon une deuxième variante, une première probabilité de bonne santé globale est déterminée par le sous-module de calcul 44 à partir de la probabilité de bonne santé pour la mesure de d'orientation de tête consolidée, de la probabilité de bonne santé pour la mesure de mouvement de tête consolidée, de la probabilité de bonne santé pour la mesure de présence consolidée, de la probabilité de bonne santé pour la mesure de fréquence de clignement oculaire consolidée et, éventuellement, de la probabilité de bonne santé pour la mesure de normalité consolidée. Les probabilités de bonne santé de ces mesures sont calculées par le sous-module de calcul 44. La première probabilité de bonne santé globale est comparée à un premier seuil de bonne santé globale prédéterminé par le sous-module de comparaison 45. Le statut de niveau de conscience prend une valeur égale à 1, indiquant une perte de conscience du membre d'équipage, si la première probabilité de bonne santé globale est inférieure ou égale au premier seuil de bonne santé globale. Sinon, le statut de niveau de conscience prend une valeur égale à 0, indiquant une non perte de conscience du membre d'équipage.

De plus, selon le mode de réalisation préféré, la troisième fonction de détection est mise en oeuvre pour les paramètres physiologiques consolidés correspondant à la mesure de fatigue, à la mesure de température corporelle, à la mesure de rythme cardiaque, à la mesure d'orientation de tête consolidée, à la mesure de mouvement de tête consolidée, à la mesure de présence consolidée, à la mesure de fréquence de clignement oculaire consolidée, à la mesure de mouvement du membre d'équipage et, éventuellement, à la mesure de normalité consolidée. Le troisième statut physiologique correspond à un statut d'incapacité du membre d'équipage.

Selon une première variante, Selon une première variante, les probabilités de bonne santé sont calculées pour chacune des mesures par le sous-module de calcul 44. La probabilité de bonne santé pour la mesure de température corporelle consolidée est comparée à un huitième seuil de bonne santé prédéterminé par le sous-module de comparaison 45. La probabilité de bonne santé pour la mesure de rythme cardiaque consolidée est comparée à un neuvième seuil de bonne santé prédéterminé par le sous-module de comparaison 45. La probabilité de bonne santé pour la mesure de d'orientation de tête consolidée est comparée à un dixième seuil de bonne santé prédéterminé par le sous-module de comparaison 45. La probabilité de bonne santé pour la mesure de mouvement de tête consolidée est comparée à un onzième seuil de bonne santé prédéterminé par le sous-module de comparaison 45. La probabilité de bonne santé pour la mesure de présence consolidée est comparée à un douzième seuil de bonne santé prédéterminé par le sous-module de comparaison 45. La probabilité de bonne santé pour la mesure de fréquence de clignement oculaire consolidée est comparée à un treizième seuil de bonne santé prédéterminé par le sous-module de comparaison 45. La probabilité de bonne santé pour la mesure de mouvement du membre d'équipage consolidée est comparée à un quatorzième seuil de bonne santé prédéterminé par le sous-module de comparaison 45. Éventuellement, la probabilité de bonne santé pour la mesure de normalité consolidée est comparée à un quinzième seuil de bonne santé prédéterminé par le sous-module de comparaison 45.

Par exemple, le statut d'incapacité prend une valeur égale à 1, indiquant une incapacité à agir du membre d'équipage :

- si la probabilité de bonne santé pour la mesure de température corporelle consolidée est supérieure ou égale au huitième seuil de bonne santé, et

- si la probabilité de bonne santé pour la mesure de rythme cardiaque consolidée est supérieure ou égale au neuvième seuil de bonne santé, et

- si la probabilité de bonne santé pour la mesure de d'orientation de tête consolidée est supérieure ou égale au dixième seuil de bonne santé prédéterminé, et

- si la probabilité de bonne santé pour la mesure de mouvement de tête consolidée est supérieure ou égale au onzième seuil de bonne santé prédéterminé, et

- si la probabilité de bonne santé pour la mesure de présence consolidée est supérieure ou égale au douzième seuil de bonne santé prédéterminé, et

- si la probabilité de bonne santé pour la mesure de fréquence de clignement oculaire consolidée est supérieure ou égale au treizième seuil de bonne santé prédéterminé, et

- si la probabilité de bonne santé pour la mesure de mouvement du membre d'équipage consolidée est supérieure ou égale au quatorzième seuil de bonne santé prédéterminé, et

- éventuellement, si la probabilité de bonne santé pour la mesure de normalité consolidée est supérieure ou égale au quinzième seuil de bonne santé prédéterminé.

Sinon, le statut d'incapacité prend une valeur égale à 0, indiquant une capacité à agir du membre d'équipage.

Selon une deuxième variante, une deuxième probabilité de bonne santé globale est déterminée par le sous-module de calcul 44 à partir de la probabilité de bonne santé pour la mesure de température corporelle consolidée, de la probabilité de bonne santé pour la mesure de rythme cardiaque consolidée, de la probabilité de bonne santé pour la mesure de d'orientation de tête consolidée, de la probabilité de bonne santé pour la mesure de mouvement de tête consolidée, de la probabilité de bonne santé pour la mesure de présence consolidée, la probabilité de bonne santé pour la mesure de fréquence de clignement oculaire consolidée, de la probabilité de bonne santé pour la mesure de mouvement du membre d'équipage consolidée et, éventuellement, de la probabilité de bonne santé pour la mesure de normalité consolidée. Les probabilités de bonne santé de ces mesures sont calculées par le sous-module de calcul 44. La deuxième probabilité de bonne santé globale est comparée à un deuxième seuil de bonne santé globale prédéterminé par le sous-module de comparaison 45. Le statut d'incapacité prend une valeur égale à 1, indiquant une incapacité à agir du membre d'équipage, si la deuxième probabilité de bonne santé globale est inférieure ou égale au deuxième seuil de bonne santé globale. Sinon, le statut d'incapacité prend une valeur égale à 0, indiquant une capacité à agir du membre d'équipage.

Le dispositif de surveillance 1 peut aussi comprendre un module de filtrage FILT (FILT pour « filtration module » en anglais) 5, configuré pour filtrer le ou les statuts physiologiques détectés par le module de fusion 4 afin de conserver le ou les statuts physiologiques les plus probables. Le but du module de filtrage 5 est de limiter les alertes parasites.

Le module de filtrage 5 est basé sur une fonction de filtrage qui peut se présenter sous la forme suivante :

$$TCS(\langle F(I), F(cs_i, cs_n)\rangle) = I',$$

dans laquelle :

- $F$ correspond à l'une des fonctions de détection,

- $I$ correspond au statut physiologique détecté par l'une des fonctions de détection,

- $cs_i$, $cs_n$ correspond aux notes de confiance les paramètres physiologiques utilisé par ladite fonction de détection pour déterminer $I$,

- $I'$ correspond au statut physiologique du membre d'équipage.

Le dispositif de filtrage peut comprendre les sous-modules suivants :

- un sous-module de calcul COMPUT2 51 configuré pour calculer une moyenne de la ou des notes de confiance pour chacune des fonctions de détection de statut physiologique, la ou les notes de confiance étant associées au ou aux modules de mesure configurés pour mesurer le ou les paramètres physiologiques utilisés par la fonction de détection de statut physiologique ;

- un sous-module de comparaison COMP3 52 configuré pour comparer la moyenne calculée par le sous-module de calcul 51 à un seuil de note de confiance prédéterminé ;

- une sous-étape de détermination DET4 53, mise en oeuvre par un quatrième sous-module de détermination, consistant à déterminer le ou les statuts physiologiques les plus probables en fonction du résultat de la comparaison par le sous-module de comparaison 52.

Le filtrage permet de rejeter les statuts physiologiques peu fiables.

La moyenne des notes de confiance peut correspondre à une moyenne arithmétique, une moyenne harmonique, une médiane, etc. Elle est définie par un expert sur la base d'expérience médicales, l'analyses de données médicales ou par un jugement d'expert.

Le seuil de filtrage peut se présenter sous la forme de règles conditionnelles, et peut être défini par un expert sur la base d'expérience médicales, l'analyses de données médicales ou par un jugement d'expert.

Le dispositif de surveillance 1 comprend aussi un module de détermination DET 6, configuré pour déterminer un niveau d'incapacité du membre de d'équipage à partir du ou des statuts physiologiques les plus probables déterminés par le module de filtrage 5.

De façon non limitative, le niveau d'incapacité peut correspondre à deux degrés d'alerte : un degré d'alerte partielle et un degré d'alerte totale. Chacun des degrés peut prendre une valeur binaire.

Le module de détermination 6 combine les statuts physiologiques filtrés pour déterminer le degré d'alerte effectif. Par exemple, un degré d'alerte est effectif quand il présente une valeur égale à 1. Sinon, il présente une valeur égale à 0.

À titre d'exemple, le degré d'alerte partielle est égal à 1 si au moins l'un des statuts physiologiques suivants présente une valeur égale à 1 : le statut de fatigue, le statut fébrile, le statut d'incapacité. Le degré d'alerte partielle est égal à 0 si tous ces statuts physiologiques présentent des valeurs égales à 0. Le degré d'alerte totale est égal à 1 si au moins l'un des statuts physiologiques suivants présente une valeur égale à 1 : le statut cardiaque, le statut de niveau de conscience, le statut d'incapacité. Le degré d'alerte totale est égal à 0 si tous ces statuts physiologiques présentent des valeurs égales à 0.

Un module de transmission TRANS (TRANS pour « transmission module » en anglais) 7 faisant partie du dispositif de surveillance 1 est configuré pour transmettre à un module utilisateur USER 8 un signal représentatif du niveau d'incapacité du membre d'équipage.

Le module utilisateur 8 peut être un dispositif d'affichage.

L"invention concerne également un procédé de surveillance de la capacité d'un membre d'équipage d'un aéronef AC.

Le procédé de surveillance comprend les étapes suivantes (figure 2) :

- une étape E1 de mesure, mise en oeuvre par le ou les modules de mesure 2, consistant à mesurer au moins un paramètre physiologique du membre d'équipage et à fournir au moins une note de confiance associée respectivement au ou aux modules de mesure 2 ;

- une étape E2 de consolidation, mise en oeuvre par le ou les modules de consolidation 3, consistant à consolider le ou les paramètres physiologiques mesurés et à déterminer la ou les notes de confiance consolidées du ou des paramètres physiologiques consolidés ;

- une étape E3 de fusion, mise en oeuvre par le module de fusion 4, consistant à fusionner le ou les paramètres physiologiques consolidés afin de détecter au moins un statut physiologique du membre d'équipage à partir d'au moins une fonction de détection de statut physiologique ;

- une étape E4 de filtrage, mise en oeuvre par le module de filtrage 5, consistant à filtrer le ou les statuts physiologiques détectés dans l'étape E3 de fusion afin de conserver le ou les statuts physiologiques les plus probables ;

- une étape E5 de détermination, mise en oeuvre par le module de détermination 6, consistant à déterminer un niveau d'incapacité du membre de d'équipage à partir du ou des statuts physiologiques les plus probables déterminés dans l'étape de filtrage ;

- une étape E6 de transmission, mise en oeuvre par le module de transmission 7, consistant à transmettre au module utilisateur 8 un signal représentatif du niveau d'incapacité du membre d'équipage.

L'étape E3 de fusion peut comprendre des sous-étapes de la première fonction de détection dont :

- une sous-étape E31 de comparaison, mise en oeuvre par le sous-module de comparaison 41, consistant à comparer au moins un paramètre physiologique consolidé par rapport à au moins un seuil d'incapacité prédéterminé ;

- une sous-étape E32 de détermination, mise en oeuvre par le sous-module de détermination 42, consistant à déterminer au moins un premier statut physiologique en fonction du résultat de la comparaison de la sous-étape E31 de comparaison.

L'étape E3 de fusion peut comprendre également des sous-étapes de la deuxième fonction de détection dont :

- une sous-étape E33 de détermination, mise en oeuvre par le sous-module de détermination 43, consistant à déterminer au moins un deuxième statut physiologique à partir d'au moins un paramètre physiologique consolidé et d'un système d'inférence comprenant des règles conditionnelles et des densités de probabilités, les règles conditionnelles et les densités de probabilité étant basées sur des expériences médicales et des analyses de données médicales.

L'étape E3 de fusion peut aussi comprendre des sous-étapes de la troisième fonction de détection dont :

- une sous-étape E34 de calcul, mise en oeuvre par le sous-module de calcul 44, consistant à calculer une probabilité de bonne santé, la probabilité de bonne santé correspondant à une probabilité de rencontrer le ou les paramètres physiologiques consolidés pour un membre d'équipage en bonne santé ;
- une sous-étape E35 de comparaison, mise en oeuvre par le sous-module de comparaison 45, consistant à comparer la probabilité de bonne santé à au moins un seuil de bonne santé prédéterminé ;
- une sous-étape E36 de détermination, mise en oeuvre par le sous-module de détermination 46, consistant à déterminer au moins un troisième statut physiologique en fonction du résultat de la comparaison de la sous-étape E35 de comparaison.

L'étape E4 de filtrage peut aussi comprendre les sous-étapes suivantes :

- une sous-étape E41 de calcul, mise en oeuvre par le sous-module de calcul 51, consistant à calculer une moyenne de la ou des notes de confiance pour chacune des fonctions de détection de statut physiologique, la ou les notes de confiance étant associées au ou aux modules de mesure 2 configurés pour mesurer le ou les paramètres physiologiques utilisés par la fonction de détection de statut physiologique ;
- une sous-étape E42 de comparaison, mise en oeuvre par le sous-module de comparaison 52, consistant à comparer la moyenne calculée dans la sous-étape E41 de calcul à un seuil de note de confiance prédéterminé ;
- une sous-étape E43 de détermination, mise en oeuvre par le sous-module de détermination 53, consistant à déterminer le ou les statuts physiologiques les plus probables en fonction du résultat de la comparaison de la sous-étape E42 de comparaison.

Selon le mode de réalisation préféré, l'étape E1 de mesure comprend les sous-étapes suivantes :

- une sous-étape E11 de mesure de fatigue, mise en oeuvre par le module de mesure de fatigue 211 disposé dans l'équipement de tête configuré pour être coiffé par le membre d'équipage et par le module de mesure de fatigue 212 disposé dans le premier équipement vidéo configuré pour acquérir des images du membre d'équipage, consistant à acquérir des mesures de fatigue du membre d'équipage ;
- une sous-étape E12 de mesure de rythme cardiaque, mise en oeuvre par le module de mesure de rythme cardiaque 221 disposé dans l'équipement de tête, le module de mesure de rythme cardiaque 222 disposé dans le premier équipement vidéo et le module de mesure de rythme cardiaque 223 disposé dans un siège configuré pour recevoir le membre d'équipage, consistant à acquérir des mesures de rythme cardiaque du membre d'équipage ;
- une sous-étape E13 de mesure de température corporelle, mise en oeuvre par le module de mesure de température corporelle 231 disposé dans l'équipement de tête, le module de mesure de température corporelle 232 disposé dans le premier équipement vidéo et le module de mesure de température corporelle 233 disposé dans le siège, consistant à acquérir des mesures de température corporelle du membre d'équipage ;
- une sous-étape E14 de mesure d'orientation de tête, mise en oeuvre par le module de mesure d'orientation de tête 241 disposé dans l'équipement de tête et le module de mesure d'orientation de tête 242 disposé dans le premier équipement vidéo, consistant à acquérir des mesures d'orientation de tête du membre d'équipage ;
- une sous-étape E15 de mesure de mouvement de tête, mise en oeuvre par le module de mesure de mouvement de tête 251 disposé dans l'équipement de tête et le module de mesure de mouvement de tête 252 disposé dans le premier équipement vidéo, consistant à acquérir des mesures de mouvement de tête du membre d'équipage ;
- une sous-étape E16 de mesure de fréquence de clignement oculaire, mise en oeuvre par le module de mesure oculaire 261 disposé dans le premier équipement vidéo, consistant à acquérir des mesures de fréquence de clignement oculaire du membre d'équipage ;
- une sous-étape E17 de mesure de présence, mise en oeuvre par le module de mesure de présence 271 disposé dans le premier équipement vidéo, consistant à acquérir des mesures de présence du membre d'équipage ;
- une sous-étape E18 de mesure de mouvement, mise en oeuvre par le module de mesure de mouvement 281 disposé dans le siège, consistant à acquérir des mesures de mouvement du membre d'équipage ;
- éventuellement, une sous-étape E19 de mesure de normalité, mise en oeuvre par le module de mesure de normalité 291 disposé dans le deuxième équipement vidéo, consistant à acquérir des mesures de normalité d'une scène dans laquelle le membre d'équipage est censé se trouver.

Selon le mode de réalisation préféré, l'étape E2 de consolidation comprend les sous-étapes suivantes :

- une sous-étape E21 de consolidation de la fatigue mesurée, mise en oeuvre par le sous-module de consolidation 31, consistant à déterminer une mesure de fatigue consolidée à partir des mesures de fatigue acquises dans la sous-étape E11 de mesure de fatigue ;
- une sous-étape E22 de consolidation du rythme cardiaque mesuré, mise en oeuvre par le sous-module de consolidation 32, consistant à déterminer une mesure de rythme cardiaque consolidée à partir des mesures de rythme cardiaque acquises dans la sous-étape E12 de mesure de rythme cardiaque ;
- une sous-étape E23 de consolidation de la température corporelle mesurée, mise en oeuvre par le sous-module de consolidation 33, consistant à déterminer une mesure de température corporelle consolidée à partir des mesures de température corporelle acquises dans la sous-étape E13 de mesure de température corporelle ;
- une sous-étape E24 de consolidation de l'orientation de tête mesurée, mise en oeuvre par le sous-module de consolidation 34, consistant à déterminer une mesure d'orientation de tête consolidée à partir des mesures d'orientation de tête acquises dans la sous-étape E14 de mesure d'orientation de tête ;
- une sous-étape E25 de consolidation du mouvement de tête mesuré, mise en oeuvre par le sous-module de consolidation 35, consistant à déterminer une mesure de mouvement de tête consolidée à partir des mesures de mouvement de tête acquises dans la sous-étape E15 de mesure de mouvement de tête.

Selon le mode de réalisation préféré, les sous-étapes E31, E32 de la première fonction de détection sont mises en oeuvre pour le paramètre physiologique consolidé correspondant à la mesure de fatigue consolidée. La mesure de fatigue consolidée est comparée au premier seuil d'incapacité prédéterminé dans la sous-étape E31 de comparaison.

Selon le mode de réalisation préféré, les sous-étapes E31, E32 de la première fonction de détection sont mises en oeuvre pour les paramètres physiologiques consolidés correspondant à la mesure de température corporelle consolidée et à la mesure de rythme cardiaque consolidée. La mesure de température corporelle consolidée est comparée au deuxième seuil d'incapacité prédéterminé dans la sous-étape E31 de comparaison. De même, la mesure de rythme cardiaque consolidée est comparée au troisième seuil d'incapacité prédéterminé dans la première sous-étape E31 de comparaison.

Selon le mode de réalisation préféré, les sous-étapes E33 de la deuxième fonction de détection sont mise en oeuvre pour les paramètres physiologiques consolidés correspondant à la mesure d'orientation de tête consolidée, à la mesure de mouvement de tête consolidée, à la mesure de présence consolidée, à la mesure de fréquence de clignement oculaire consolidée et, éventuellement, à la mesure de normalité consolidée.

Selon le mode de réalisation préféré, les sous-étapes E34, E35, E36 de la troisième fonction de détection sont mises en oeuvre pour les paramètres physiologiques consolidés correspondant à la mesure de température corporelle consolidée et à la mesure de rythme cardiaque consolidée. La probabilité de bonne santé pour la mesure de température corporelle consolidée est comparée au premier seuil de bonne santé prédéterminé dans la sous-étape E35 de comparaison. La probabilité de bonne santé pour la mesure de rythme cardiaque consolidée est comparée au deuxième seuil de bonne santé prédéterminé dans la sous-étape E35 de comparaison.

Selon une première variante du mode de réalisation préféré, les sous-étapes E34, E35, E36 de la troisième fonction de détection sont mises en oeuvre pour les paramètres physiologiques consolidés correspondant à la mesure d'orientation de tête consolidée, à la mesure de mouvement de tête consolidée, à la mesure de présence consolidée, à la mesure de fréquence de clignement oculaire consolidée et à la mesure de normalité consolidée. La probabilité de bonne santé pour la mesure de d'orientation de tête consolidée est comparée au troisième seuil de bonne santé prédéterminé dans la sous-étape E35 de comparaison. La probabilité de bonne santé pour la mesure de mouvement de tête consolidée est comparée au quatrième seuil de bonne santé prédéterminé dans la sous-étape E35 de comparaison. La probabilité de bonne santé pour la mesure de présence consolidée est comparée au cinquième seuil de bonne santé prédéterminé dans la sous-étape E35 de comparaison. La probabilité de bonne santé pour la mesure de fréquence de clignement oculaire consolidée est comparée dans la sous-étape E35 de comparaison au sixième seuil de bonne santé prédéterminé. Éventuellement, la probabilité de bonne santé pour la mesure de normalité consolidée est comparée dans la sous-étape E35 de comparaison au septième seuil de bonne santé prédéterminé.

Selon une deuxième variante du mode de réalisation préféré, les sous-étapes E34, E35, E36 de la troisième fonction de détection sont mises en oeuvre pour les paramètres physiologiques consolidés correspondant à la mesure d'orientation de tête consolidée, à la mesure de mouvement de tête consolidée, à la mesure de présence consolidée, à la mesure de fréquence de clignement oculaire consolidée et, éventuellement, à la mesure de normalité consolidée. La première probabilité de bonne santé globale est déterminée dans la sous-étape E34 à partir de la probabilité de bonne santé pour la mesure de d'orientation de tête consolidée, de la probabilité de bonne santé pour la mesure de mouvement de tête consolidée, de la probabilité de bonne santé pour la mesure de présence consolidée, de la probabilité de bonne santé pour la mesure de fréquence de clignement oculaire consolidée et, éventuellement, de la probabilité de bonne santé pour la mesure de normalité consolidée. La première probabilité de bonne santé globale est comparée à au premier seuil de bonne santé globale prédéterminé dans la sous-étape E35.

Selon une première variante du mode de réalisation préféré, les sous-étapes E34, E35, E36 de la troisième fonction de détection sont mises en oeuvre pour les paramètres physiologiques consolidés correspondant à la mesure de fatigue, à la mesure de température corporelle, à la mesure de rythme cardiaque, à la mesure d'orientation de tête consolidée, à la mesure de mouvement de tête consolidée, à la mesure de présence consolidée, à la mesure de fréquence de clignement oculaire consolidée, à la mesure de mouvement du membre d'équipage et, éventuellement, à la mesure de normalité consolidée. La probabilité de bonne santé pour la mesure de température corporelle consolidée est comparée au huitième seuil de bonne santé prédéterminé dans la sous-étape E35 de comparaison. La probabilité de bonne santé pour la mesure de rythme cardiaque consolidée est comparée au neuvième seuil de bonne santé prédéterminé dans la sous-étape E35 de comparaison. La probabilité de bonne santé pour la mesure de d'orientation de tête consolidée est comparée dans la sous-étape E35 de comparaison au dixième seuil de bonne santé prédéterminé. La probabilité de bonne santé pour la mesure de mouvement de tête consolidée est comparée au onzième seuil de bonne santé prédéterminé dans la sous-étape E35 de comparaison. La probabilité de bonne santé pour la mesure de présence consolidée est comparée dans la sous-étape E35 de comparaison au douzième seuil de bonne santé prédéterminé. La probabilité de bonne santé pour la mesure de fréquence de clignement oculaire consolidée est comparée au treizième seuil de bonne santé prédéterminé dans la sous-étape E35 de comparaison. La probabilité de bonne santé pour la mesure de mouvement du membre d'équipage consolidée est comparée au quatorzième seuil de bonne santé prédéterminé dans la sous-étape E35 de comparaison. Éventuellement, la probabilité de bonne santé pour la mesure de normalité consolidée est comparée au quinzième seuil de bonne santé prédéterminé dans la sous-étape E35 de comparaison.

Selon une deuxième variante du mode de réalisation préféré, les sous-étapes E34, E35, E36 de la troisième fonction de détection sont mises en oeuvre pour les paramètres physiologiques consolidés correspondant à la mesure de fatigue, à la mesure de température corporelle, à la mesure de rythme cardiaque, à la mesure d'orientation de tête consolidée, à la mesure de mouvement de tête consolidée, à la mesure de présence consolidée, à la mesure de fréquence de clignement oculaire consolidée, à la mesure de mouvement du membre d'équipage et, éventuellement, à la mesure de normalité consolidée. La deuxième probabilité de bonne santé globale est déterminée dans la sous-étape E34 à partir de la probabilité de bonne santé pour la mesure de température corporelle consolidée, de la probabilité de bonne santé pour la mesure de rythme cardiaque consolidée, de la probabilité de bonne santé pour la mesure de d'orientation de tête consolidée, de la probabilité de bonne santé pour la mesure de mouvement de tête consolidée, de la probabilité de bonne santé pour la mesure de présence consolidée, de la probabilité de bonne santé pour la mesure de fréquence de clignement oculaire consolidée, de la probabilité de bonne santé pour la mesure de mouvement du membre d'équipage consolidée et, éventuellement, de la probabilité de bonne santé pour la mesure de normalité consolidée. La deuxième probabilité de bonne santé globale est comparée dans la sous-étape E35 au deuxième seuil de bonne santé globale prédéterminé. Le statut d'incapacité prend une valeur égale à 1, indiquant une incapacité à agir du membre d'équipage, si la deuxième probabilité de bonne santé globale est inférieure ou égale au deuxième seuil de bonne santé globale. Sinon, le statut d'incapacité prend une valeur égale à 0, indiquant une capacité à agir du membre d'équipage.

## Revendications

1. Procédé de surveillance de la capacité d'un membre d'équipage d'un aéronef (AC),
   **caractérisé en ce qu'**il comprend les étapes suivantes :

   - une étape (E1) de mesure, mise en oeuvre par au moins un module de mesure (2), consistant à mesurer au moins un paramètre physiologique du membre d'équipage et à fournir au moins une note de confiance associée respectivement au ou aux modules de mesure (2) ;
   - une étape (E2) de consolidation, mise en oeuvre par au moins un module de consolidation (3), consistant à consolider le ou les paramètres physiologiques mesurés et à déterminer une ou des notes de confiance consolidées du ou des paramètres physiologiques consolidés ;
   - une étape (E3) de fusion, mise en oeuvre par un module de fusion (4), consistant à fusionner le ou les paramètres physiologiques consolidés afin de détecter au moins un statut physiologique du membre d'équipage à partir d'au moins une fonction de détection de statut physiologique ;
   - une étape (E4) de filtrage, mise en oeuvre par un module de filtrage (5), consistant à filtrer le ou les statuts physiologiques détectés dans l'étape (E3) de fusion afin de conserver le ou les statuts physiologiques les plus probables ;
   - une étape (E5) de détermination, mise en oeuvre par un module de détermination (6), consistant à déterminer un niveau d'incapacité du membre de d'équipage à partir du ou des statuts physiologiques les plus probables déterminés dans l'étape (E4) de filtrage ;

- une étape (E6) de transmission, mise en oeuvre par un module de transmission (7), consistant à transmettre à un module utilisateur un signal représentatif du niveau d'incapacité du membre d'équipage.

**2.** Procédé selon la revendication 1, **caractérisé en ce que** l'étape (E3) de fusion comprend des sous-étapes d'une première fonction de détection dont :

- une première sous-étape (E31) de comparaison, mise en oeuvre par un premier sous-module de comparaison (41), consistant à comparer au moins un paramètre physiologique consolidé par rapport à au moins un seuil d'incapacité prédéterminé ;
- une première sous-étape (E32) de détermination, mise en oeuvre par un premier sous-module de détermination (42), consistant à déterminer au moins un premier statut physiologique en fonction du résultat de la comparaison de la première sous-étape (E31) de comparaison.

**3.** Procédé selon l'une quelconque des revendications 1 ou 2, **caractérisé en ce que** l'étape (E3) de fusion comprend des sous-étapes d'une deuxième fonction de détection dont :

- une deuxième sous-étape (E33) de détermination, mise en oeuvre par un deuxième sous-module de détermination (43), consistant à déterminer au moins un deuxième statut physiologique à partir d'au moins un paramètre physiologique consolidé et d'un système d'inférence comprenant des règles conditionnelles et des densités de probabilités, les règles conditionnelles et les densités de probabilité étant basées sur des expériences médicales et des analyses de données médicales.

**4.** Procédé selon l'une quelconque des revendications 1 à 3, **caractérisé en ce que** l'étape (E3) de fusion comprend des sous-étapes d'une troisième fonction de détection dont :

- une première sous-étape (E34) de calcul, mise en oeuvre par un premier sous-module de calcul (44), consistant à calculer une probabilité de bonne santé, la probabilité de bonne santé correspondant à une probabilité de rencontrer le ou les paramètres physiologiques consolidés pour un membre d'équipage en bonne santé ;

- une deuxième sous-étape (E35) de comparaison, mise en oeuvre par un deuxième sous-module de comparaison (45), consistant à comparer la probabilité de bonne santé à au moins un seuil de bonne santé prédéterminé ;
- une troisième sous-étape (E36) de détermination, mise en oeuvre par un troisième sous-module de détermination (46), consistant à déterminer au moins un troisième statut physiologique en fonction du résultat de la comparaison de la deuxième sous-étape (E35) de comparaison.

**5.** Procédé selon l'une quelconque des revendications 1 à 4, **caractérisé en ce que** l'étape (E4) de filtrage comprend les sous-étapes suivantes :

- une deuxième sous-étape (E41) de calcul, mise en oeuvre par un deuxième sous-module de calcul (51), consistant à calculer une moyenne de la ou des notes de confiance pour chacune des fonctions de détection de statut physiologique, la ou les notes de confiance étant associées au ou aux modules de mesure (2) configurés pour mesurer le ou les paramètres physiologiques utilisés par la fonction de détection de statut physiologique ;
- une troisième sous-étape (E42) de comparaison, mise en oeuvre par un troisième sous-module de comparaison (52), consistant à comparer la moyenne calculée dans la deuxième sous-étape (E41) de calcul à un seuil de note de confiance prédéterminé ;
- une quatrième sous-étape (E43) de détermination, mise en oeuvre par un quatrième sous-module de détermination (53), consistant à déterminer le ou les statuts physiologiques les plus probables en fonction du résultat de la comparaison de la troisième sous-étape (E42) de comparaison.

**6.** Procédé selon l'une quelconque des revendications 1 à 5, **caractérisé en ce que** l'étape (E1) de mesure comprend les sous-étapes suivantes :

- une sous-étape (E11) de mesure de fatigue, mise en oeuvre par un premier module de mesure de fatigue (211) disposé dans un équipement de tête configuré pour être coiffé par le membre d'équipage et par un deuxième module de mesure de fatigue (212) disposé dans un premier équipement vidéo configuré pour acquérir des images du membre d'équipage, consistant à acquérir des mesures de fatigue du membre d'équipage ;
- une sous-étape (E12) de mesure de rythme cardiaque, mise en oeuvre par un premier module de mesure de rythme cardiaque (221) disposé dans l'équipement de tête, un deuxième module de mesure de rythme cardiaque (222) disposé dans le premier équipement vidéo et un troisième module de mesure de rythme cardiaque (223) disposé dans un siège configuré pour recevoir le membre d'équipage, consistant à acquérir des mesures de rythme cardiaque du membre d'équipage ;
- une sous-étape (E13) de mesure de température corporelle, mise en oeuvre par un premier module de mesure de température corporelle (231) disposé dans l'équipement de tête, un deuxième module de mesure de température corporelle (232) disposé dans le premier équipement vidéo et un troisième module de mesure de température corporelle (233) disposé dans le siège, consistant à acquérir des mesures de température corporelle du membre d'équipage ;
- une sous-étape (E14) de mesure d'orientation de tête, mise en oeuvre par un premier module de mesure d'orientation de tête (241) disposé dans l'équipement de tête et un deuxième module de mesure d'orientation de tête (242) disposé dans le premier équipement vidéo, consistant à acquérir des mesures d'orientation de tête du membre d'équipage ;
- une sous-étape (E15) de mesure de mouvement de tête, mise en oeuvre par un premier module de mesure de mouvement de tête (251) disposé dans l'équipement de tête et un deuxième module de mesure de mouvement de tête (252) disposé dans le premier équipement vidéo, consistant à acquérir des mesures de mouvement de tête du membre d'équipage ;
- une sous-étape (E16) de mesure de fréquence de clignement oculaire, mise en oeuvre par un module de mesure oculaire (261) disposé dans le premier équipement vidéo, consistant à acquérir des mesures de fréquence de clignement oculaire du membre d'équipage ;
- une sous-étape (E17) de mesure de présence, mise en oeuvre par un module de mesure de présence (271) disposé dans le premier équipement vidéo, consistant à acquérir des mesures de présence du membre d'équipage ;
- une sous-étape (E18) de mesure de mouvement, mise en oeuvre par un module de mesure de mouvement (281) disposé dans le siège, consistant à acquérir des mesures de mouvement du membre d'équipage.

7. Procédé selon la revendication 6,
**caractérisé en ce que** l'étape (E2) de consolidation comprend les sous-étapes suivantes :

- une sous-étape (E21) de consolidation de la fatigue mesurée, mise en oeuvre par un premier sous-module de consolidation (31), consistant à déterminer une mesure de fatigue consolidée à partir des mesures de fatigue acquises dans la sous-étape (E11) de mesure de fatigue ;
- une sous-étape (E22) de consolidation du rythme cardiaque mesuré, mise en oeuvre par un deuxième sous-module de consolidation (32), consistant à déterminer une mesure de rythme cardiaque consolidée à partir des mesures de rythme cardiaque acquises dans la sous-étape (E12) de mesure de rythme cardiaque ;
- une sous-étape (E23) de consolidation de la température corporelle mesurée, mise en oeuvre par un troisième sous-module de consolidation (33), consistant à déterminer une mesure de température corporelle consolidée à partir des mesures de température corporelle acquises dans la sous-étape (E13) de mesure de température corporelle ;
- une sous-étape (E24) de consolidation de l'orientation de tête mesurée, mise en oeuvre par un quatrième sous-module de consolidation (34), consistant à déterminer une mesure d'orientation de tête consolidée à partir des mesures d'orientation de tête acquises dans la sous-étape (E14) de mesure d'orientation de tête ;
- une sous-étape (E25) de consolidation du mouvement de tête mesuré, mise en oeuvre par un cinquième sous-module de consolidation (35), consistant à déterminer une mesure de mouvement de tête consolidée à partir des mesures de mouvement de tête acquises dans la sous-étape (E15) de mesure de mouvement de tête.

8. Procédé selon l'une quelconque des revendications 6 ou 7,
**caractérisé en ce que** les sous-étapes de la première fonction de détection sont mises en oeuvre pour le paramètre physiologique consolidé correspondant à la mesure de fatigue consolidée,
le premier statut physiologique correspondant à un statut de fatigue,
la mesure de fatigue consolidée étant comparée à un premier seuil d'incapacité prédéterminé dans la première sous-étape (E31) de comparaison.

9. Procédé selon l'une quelconque des revendications 6 à 8,
**caractérisé en ce que** les sous-étapes de la première fonction de détection sont mises en oeuvre pour les paramètres physiologiques consolidés correspondant à la mesure de température corporelle consolidée et à la mesure de rythme cardiaque consolidée,
les premiers statuts physiologiques correspondant à un statut fébrile et à un statut cardiaque,

la mesure de température corporelle consolidée étant comparée à un deuxième seuil d'incapacité prédéterminé dans la première sous-étape (E31) de comparaison,

la mesure de rythme cardiaque consolidée étant comparée à un troisième seuil d'incapacité prédéterminé dans la première sous-étape (E31) de comparaison.

**10.** Procédé selon l'une quelconque des revendications 6 à 8,

**caractérisé en ce que** les sous-étapes de la troisième fonction de détection sont mises en oeuvre pour les paramètres physiologiques consolidés correspondant à la mesure de température corporelle consolidée et à la mesure de rythme cardiaque consolidée,

les troisièmes statuts physiologiques correspondant à un statut fébrile et à un statut cardiaque,

la probabilité de bonne santé pour la mesure de température corporelle consolidée étant comparée à un premier seuil de bonne santé prédéterminé dans la deuxième sous-étape (E35) de comparaison,

la probabilité de bonne santé pour la mesure de rythme cardiaque consolidée étant comparée à un deuxième seuil de bonne santé prédéterminé dans la deuxième sous-étape (E35) de comparaison.

**11.** Procédé selon l'une quelconque des revendications 6 à 10,

**caractérisé en ce que** les sous-étapes de la deuxième fonction de détection sont mise en oeuvre pour les paramètres physiologiques consolidés correspondant à la mesure d'orientation de tête consolidée, à la mesure de mouvement de tête consolidée, à la mesure de présence consolidée et à la mesure de fréquence de clignement oculaire consolidée,

le deuxième statut physiologique correspondant à un premier statut de niveau de conscience.

**12.** Procédé selon l'une quelconque des revendications 6 à 10,

**caractérisé en ce que** les sous-étapes de la troisième fonction de détection sont mises en oeuvre pour les paramètres physiologiques consolidés correspondant à la mesure d'orientation de tête consolidée, à la mesure de mouvement de tête consolidée, à la mesure de présence consolidée et à la mesure de fréquence de clignement oculaire consolidée,

le troisième statut physiologique correspondant à un deuxième statut de niveau de conscience,

une première probabilité de bonne santé globale étant déterminée dans la première sous-étape (E34) de calcul à partir d'une probabilité de bonne santé pour la mesure de d'orientation de tête consolidée, d'une probabilité de bonne santé pour la mesure de mouvement de tête consolidée, d'une probabilité de bonne santé pour la mesure de présence consolidée et d'une probabilité de bonne santé pour la mesure de fréquence de clignement oculaire consolidée, la première probabilité de bonne santé globale étant comparée dans la deuxième sous-étape (E35) de comparaison à un premier seuil de bonne santé globale prédéterminé.

**13.** Procédé selon l'une quelconque des revendications 6 à 12,

**caractérisé en ce que** les sous-étapes de la troisième fonction de détection sont mises en oeuvre pour les paramètres physiologiques consolidés correspondant à la mesure de fatigue, à la mesure de température corporelle, à la mesure de rythme cardiaque, à la mesure d'orientation de tête consolidée, à la mesure de mouvement de tête consolidée, à la mesure de présence consolidée, à la mesure de fréquence de clignement oculaire consolidée et à la mesure de mouvement du membre d'équipage,

le troisième statut physiologique correspondant à un statut d'incapacité du membre d'équipage,

une deuxième probabilité de bonne santé globale est déterminée dans la première sous-étape (E34) de calcul à partir d'une probabilité de bonne santé pour la mesure de température corporelle consolidée, d'une probabilité de bonne santé pour la mesure de rythme cardiaque consolidée, d'une probabilité de bonne santé pour la mesure de d'orientation de tête consolidée, d'une probabilité de bonne santé pour la mesure de mouvement de tête consolidée, d'une probabilité de bonne santé pour la mesure de présence consolidée, d'une probabilité de bonne santé pour la mesure de fréquence de clignement oculaire consolidée et d'une probabilité de bonne santé pour la mesure de mouvement du membre d'équipage consolidée,

la deuxième probabilité de bonne santé globale étant comparée dans la deuxième étape (E35) de comparaison à un deuxième seuil de bonne santé globale prédéterminé.

**14.** Dispositif de surveillance de la capacité d'un membre d'équipage d'un aéronef (AC),

**caractérisé en ce qu'**il comprend :

- au moins un module de mesure (2), configuré pour mesurer au moins un paramètre physiologique du membre d'équipage et à fournir au moins une note de confiance associée respectivement au ou aux modules de mesure (2) ;
- au moins un module de consolidation (3), configuré pour consolider le ou les paramètres physiologiques mesurés et à déterminer la ou les notes de confiance consolidées du ou des paramètres physiologiques consolidés ;
- un module de fusion (4), configuré pour fusionner le ou les paramètres physiologiques consolidés afin de détecter au moins un statut physiologique du membre d'équipage à partir d'au moins une fonction de détection de statut physiologique ;

- un module de filtrage (5), configuré pour filtrer le ou les statuts physiologiques détectés par le module de fusion (4) afin de conserver le ou les statuts physiologiques les plus probables ;

- un module de détermination (6), configuré pour déterminer un niveau d'incapacité du membre de d'équipage à partir du ou des statuts physiologiques les plus probables déterminés par le module de filtrage (5) ;

- un module de transmission (7), configuré pour transmettre à un module utilisateur (8) un signal représentatif du niveau d'incapacité du membre d'équipage.

15. Aéronef,
**caractérisé en ce qu'**il comporte un dispositif de surveillance (1) de la capacité d'un membre d'équipage d'un aéronef (AC), tel que celui spécifié sous la revendication 14.

Fig. 1

MEAS

MEAS1 — 211
MEAS2 — 212

MEAS3 — 221
MEAS4
MEAS5 — 223
222

MEAS6 — 231
MEAS7
MEAS8 — 233
232

MEAS9 — 241
MEAS10
242

MEAS11 — 251
MEAS12
252

MEAS13 — 261

MEAS14 — 271

MEAS15 — 281

MEAS16 — 291

— 2

CONS

CONS1 — 31

CONS2 — 32

CONS3 — 33

CONS4 — 34

CONS5 — 35

— 3

FUSION

COMP1 — 41

DET1 — 42

DET2
43

COMPUT1 — 44

COMP2 — 45

DET3 — 46

— 4
— 1

FILT

COMPUT2 — 51

COMP3 — 52

DET4 — 53

5

DET — 6

TRANS — 7

USER — 8

Fig. 2

AC

1

Fig. 3

Europäisches
Patentamt

European
Patent Office

Office européen
des brevets

**RAPPORT DE RECHERCHE EUROPEENNE**

Numéro de la demande

EP 19 18 1383

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | US 2017/332975 A1 (PAPAIX BENOIT [FR] ET AL) 23 novembre 2017 (2017-11-23)<br>* figures 1-3, 4a-d *<br>* alinéa [0069] - alinéa [0122] *<br>----- | 1-15 | INV.<br>A61B5/01<br>A61B5/11<br>A61B5/18<br>A61B5/00 |
| A | EP 3 243 430 A1 (IMEC VZW [BE]; UNIV LEUVEN KATH [BE]) 15 novembre 2017 (2017-11-15)<br>* figure 1 *<br>* alinéa [0061] - alinéa [0085] *<br>----- | 1-15 | A61B5/16<br>G16H50/00<br>B60K28/02<br><br>ADD.<br>A61B5/0402 |
| A | WO 2017/108548 A1 (KONINKLIJKE PHILIPS NV [NL]) 29 juin 2017 (2017-06-29)<br>* page 8, ligne 11 - page 18, ligne 2 *<br>----- | 1-15 | A61B5/0476<br>A61B5/08 |
| A | US 2008/122636 A1 (MATOS JEFFREY A [US]) 29 mai 2008 (2008-05-29)<br>* figure 1 *<br>----- | 1-15 | |
| A | US 7 027 621 B1 (PROKOSKI FRANCINE J [US]) 11 avril 2006 (2006-04-11)<br>* figures 7, 10, 12, 15-17 *<br>* colonne 7, ligne 6 - colonne 8, ligne 46 *<br>----- | 1-15 | DOMAINES TECHNIQUES RECHERCHES (IPC)<br><br>A61B<br>G06N |
| A | US 2015/257681 A1 (SHUSTER GARY STEPHEN [US] ET AL) 17 septembre 2015 (2015-09-17)<br>* figures 1-4 *<br>* alinéa [0043] - alinéa [0067] *<br>* alinéa [0082] - alinéa [0083] *<br>----- | 1-15 | G16H<br>B60K<br>G06F |

Le présent rapport a été établi pour toutes les revendications

| Lieu de la recherche | Date d'achèvement de la recherche | Examinateur |
|---|---|---|
| La Haye | 8 juillet 2019 | Knoop, Jan |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
................................................................
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 19 18 1383

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

08-07-2019

| Document brevet cité au rapport de recherche | | Date de publication | Membre(s) de la famille de brevet(s) | | Date de publication |
|---|---|---|---|---|---|
| US 2017332975 | A1 | 23-11-2017 | FR<br>US | 3051342 A1<br>2017332975 A1 | 24-11-2017<br>23-11-2017 |
| EP 3243430 | A1 | 15-11-2017 | EP<br>US | 3243430 A1<br>2017325701 A1 | 15-11-2017<br>16-11-2017 |
| WO 2017108548 | A1 | 29-06-2017 | CN<br>EP<br>JP<br>US<br>WO | 108430321 A<br>3393348 A1<br>2019503749 A<br>2018360387 A1<br>2017108548 A1 | 21-08-2018<br>31-10-2018<br>14-02-2019<br>20-12-2018<br>29-06-2017 |
| US 2008122636 | A1 | 29-05-2008 | AUCUN | | |
| US 7027621 | B1 | 11-04-2006 | AUCUN | | |
| US 2015257681 | A1 | 17-09-2015 | US<br>US<br>US | 2015257681 A1<br>2015262429 A1<br>2018296134 A1 | 17-09-2015<br>17-09-2015<br>18-10-2018 |

EPO FORM P0460

Pour tout renseignement concernant cette annexe : voir Journal Officiel de l'Office européen des brevets, No.12/82

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2017332975 A